# FASCICULE DE BREVET EUROPEEN

(11) **EP 1 194 772 B1**
(45) Date de publication et mention de la délivrance du brevet: **02.04.2003**
(21) Numéro de dépôt: 00949639.9
(22) Date de dépôt: 05.07.2000
(51) Int. Cl.: G01N 29/02, G01N 33/20, G01N 15/02

(54) **PROCEDE ET DISPOSITIF DE COMPTAGE DES INCLUSIONS DANS UN BAIN DE METAL LIQUIDE PAR ULTRASONS**
VERFAHREN UND VORRICHTUNG ZUM ZÄHLEN VON EINSCHLÜSSEN IN EINER IN EINEM GEFÄSS BEFINDLICHEN METALLSCHMELZE MITTELS ULTRASCHALL
IMPROVED METHOD AND DEVICE FOR COUNTING INCLUSIONS IN A LIQUID METAL BATH WITH ULTRASOUNDS

(30) Priorité: 09.07.1999 FR 9909123
(43) Date de publication de la demande: 10.04.2002
(73) Titulaire: Pechiney Rhenalu, 75116 Paris (FR); ALUMINIUM PECHINEY, 75218 Paris Cedex 16 (FR)
(72) Inventeur: MULLER, Jean, F-09400 Tarascon sur Ariege (FR); LE BRUN, Pierre, F-38110 Saint-Jean-de-Soudain (FR); ODIEVRE, Thierry, F-38600 Coublevie (FR)
(74) Mandataire: Marsolais, Richard
(86) Numéro de dépôt international: FR0001927
(87) Numéro de publication internationale: WO01004620

(56) Documents cités:
- WO-A-98/46987
- US-A- 4 843 866
- US-A- 5 708 209

## Description

### Domaine technique

L'invention concerne un procédé et un dispositif améliorés à ultrasons permettant de compter les inclusions liquides et/ou solides présentes dans un écoulement de métal liquide circulant généralement dans une cuve ou mieux une goulotte, et de mesurer leur taille. L'invention est applicable en particulier à l'aluminium, au magnésium ou à leurs alliages.

### Etat de la technique

Dans le domaine de la coulée de l'aluminium liquide il est de la plus haute importance de contrôler de façon précise la qualité inclusionaire; d'elle, en effet, dépend la qualité et le taux de mise au rebuts des tôles minces, en particulier celles obtenues lors de la fabrication des récipients fermés du type boîte de boisson ou aérosol.

Généralement la qualité inclusionaire est déterminée par le taux d'inclusions contenues dans un métal liquide et par leur taille.

Le plus souvent les méthodes de caractérisation partent de prélèvements permettant d'échantillonner au mieux environ 0,01 % du métal liquide. Compte tenu que le niveau du taux d'inclusions à mesurer est faible (de l'ordre de la ppm), qu'il y a peu d'inclusions de grosse taille et que ces dernières sont les plus nuisibles dès que l'on veut obtenir une tôle mince, on voit que ces méthodes par prélèvements présentent le risque de donner des résultats peu représentatifs avec une très faible probabilité de voir les dites inclusions.

A titre d'illustration on connaît une méthode, dite Podfa (Porous Disk Filtration of Aluminium), consistant essentiellement à prélever un échantillon de métal liquide de façon discontinue, à le filtrer et à analyser les inclusions récupérées; mais cette méthode est seulement indicative en taille et en nombre d'inclusions. On connaît également une méthode, dite LIMCA (Liquid Metal Cleanliness Analysis), consistant essentiellement à échantillonner le métal liquide en continu à travers un petit orifice et à y mesurer la variation de résistance du métal liquide à chaque passage d'inclusion; mais cette méthode ne fonctionne pas pour les grosses inclusions (c'est-à-dire des inclusions dont la taille est supérieure à environ 100 µm). Il apparaît donc difficile de bien mesurer, à l'aide de ces méthodes, les progrès qui peuvent être encore faits dans l'élimination des inclusions en particulier celles de grosse taille.

La qualité inclusionaire peut aussi être évaluée par des mesures faites in situ et en continu à l'aide d'ultrasons; un tel type de méthode peut permettre d'échantillonner jusqu'à 5 % du métal liquide, en particulier quand celui- ci circule dans une goulotte.

Par exemple dans le brevet US 4981045 (University of Toronto) il est décrit une sonde à ultrasons et un procédé mis en oeuvre pour tester la présence de défauts, en particulier d'inclusions, dans un creuset de métal liquide. Cette sonde comporte une ligne à retard, faisant la liaison entre le cristal piézoélectrique émetteur-récepteur et le métal liquide, fusible dans ce dernier; pour cela elle est généralement à base du même métal que le métal à analyser et est refroidie pour éviter sa destruction complète. Il y a alors continuité acoustique entre la dite ligne à retard et le dit métal liquide.

La méthode consiste essentiellement à immerger dans le dit métal liquide deux sondes par l'intermédiaire de l'extrémité libre de leur ligne à retard; des trains d'onde sont régulièrement émis et les signaux réfléchis sont analysés. On recueille un pic important dû à l'écho des ultrasons sur la paroi du creuset; une teneur moyenne en inclusions de la fraction de métal analysée est déduite de l'affaiblissement de ce pic, un étalonnage du dit pic étant préalablement fait à l'aide d'un métal réputé pur.

Il est aussi fait mention que l'on peut compter le nombre d'inclusions se trouvant à une certaine profondeur dans le volume testé par les sondes en comptant le nombre de pics dus aux réflexions du signal sur les inclusions à l'aide d'un ordinateur et qu'il est difficile de faire des mesures dans tout le métal contenu dans le creuset. Une telle méthode n'est pas étalonnée et le comptage pas précis.

Le brevet US 5708209 (Alcoa) décrit également l'emploi d'un type particulier de sondes, émettrice et réceptrice, avec des lignes à retard fusibles, pour détecter les particules présentes dans un métal liquide. Les mêmes remarques que précédemment peuvent être faites.

Ces procédés permettent de n'analyser qu'un volume restreint de métal liquide, ne donnent généralement que des renseignements globaux de type indicatif sur les inclusions permettant seulement de dire si le métal liquide est propre ou sale et ne renseigne pas de façon précise sur le nombre et la taille des inclusions.

Des tentatives ont été faites pour essayer d'avoir des renseignements sur la taille des inclusions en introduisant dans de l'aluminium liquide réputé pur des particules de taille connue et en comparant le comptage obtenu avec celui effectué sur le métal à analyser. Une telle méthode n'est pas utilisable industriellement. En effet il apparaît illusoire de vouloir faire passer du métal pur dans une installation industrielle sans risque de le souiller, la mesure d'étalonnage se trouvant de ce fait biaisée; de plus l'introduction de particules est de nature à polluer et perturber fortement le cycle industriel de fabrication.

Par ailleurs pour avoir des renseignements précis sur la taille il faudrait que les particules soient parfaitement sphériques et que la taille des particules contenues dans la coupe granulométrique introduite dans le métal pur soit homogène, ce qui n'est pas habituellement le cas. D'autre part les particules peuvent se faire de l'ombre et s'agglomérer. Tout ceci ne rend pas la méthode fiable et n'est pas simple à réaliser.

Ainsi la demanderesse a cherché une méthode fiable et donnant des résultats significatifs qui permette d'analyser la qualité inclusionaire d'un métal liquide. Elle a en particulier cherché une méthode pour compter et donner des renseignements précis sur la taille des inclusions en particulier des grosses inclusions (typiquement supérieures à 50 µm) même en faible quantité, méthode qui soit calibrée, et dont l'exactitude puisse être contrôlée périodiquement sans dérégler le déroulement des opérations industrielles.

### Description de l'invention

L'invention est un procédé de visualisation, de mesure de la taille et de comptage individuels des inclusions en suspension dans un métal liquide en mouvement, à l'aide d'un capteur comprenant au moins un moyen pour émettre une succession de tirs de faisceaux d'ultrasons au sein du dit métal liquide, au moins un moyen pour recevoir les échos réfléchis par les dites inclusions, et leurs accessoires, caractérisé en ce qu'il comporte une étape d'étalonnage de la réponse du capteur, la dite étape mettant en oeuvre au moins un réflecteur étalon de dimension connue stable au cours du temps, des étapes successives d'acquisition et de traitement des échos réfléchis, une étape de visualisation, par exemple de type B scan comme cela sera vu plus loin, et un étape d'analyse d'image pour compter et mesurer le diamètre des inclusions.

En d'autres termes, le procédé selon l'invention comprend l'émission d'une succession de tirs de faisceaux d'ultrasons au sein du dit métal liquide à l'aide dudit capteur, la réception des échos réfléchis par les dites inclusions à l'aide dudit capteur, une étape d'étalonnage de la réponse dudit capteur, des étapes successives d'acquisition et de traitement des échos réfléchis, une étape de visualisation, par exemple de type B scan, et un étape d'analyse d'image pour compter et mesurer le diamètre des inclusions.

Bien que le procédé puisse être mis en oeuvre dans tout type de cuve de traitement de métal liquide, il est particulièrement intéressant de l'utiliser dans des goulottes de circulation du dit métal où il permet d'en analyser en continu une proportion importante voire la totalité.

Pour l'étalonnage il est important d'avoir au moins un, et de préférence une pluralité de, réflecteur(s) étalon(s) de taille connue individuellement, stable, de géométrie connue et maîtrisée, donnant un écho reproductible au cours du temps. Dans le mode de réalisation préféré de l'invention, on utilise une pluralité de réflecteurs de taille différente. Il est ainsi possible d'établir une courbe d'étalonnage de la réponse du capteur donnant l'amplitude du signal d'écho en fonction des dites dimensions des réflecteurs étalons et d'extrapoler la courbe pour des inclusions de petite taille.

Les réflecteurs peuvent être avantageusement constitués par la section droite d'une ou plusieurs tiges ayant un diamètre calibré (et donc connu). Les tiges sont de préférence inertes, c'est-à-dire qu'elles sont de préférence en matériau inerte vis-à-vis du métal liquide, tel qu'une céramique réfractaire. Les tiges, fixées généralement sur un support déplaçable, sont immergées typiquement dans le champ du capteur dans le métal liquide pendant l'étape d'étalonnage, puis retirées hors de son champ pendant les séquences d'analyse du métal liquide; elles peuvent y être réintroduites périodiquement pour vérifier le dit étalonnage. Il est généralement suffisant que seule une extrémité de la ou des tiges soit immergée dans le champ du capteur. L'extrémité de la ou des tige(s) immergée dans le champ du capteur (et plus précisément dans la tache focale formée par l'intersection des faisceaux d'ultrasons émis et reçus par lesdits moyens d'émission et de réception), qui peut avoir différentes géométries, est avantageusement à face plane afin d'obtenir une réflexion optimale. Cette extrémité est typiquement l'extrémité supérieure de la tige lorsque cette dernière est disposée verticalement. On utilise de préférence une pluralité de tiges de diamètre différent, ce qui permet d'établir efficacement une courbe d'étalonnage du capteur en fonction de la taille des inclusions. D'autres types de réflecteurs étalons peuvent être utilisés par exemple des sphères, des cônes ou des cavités calibrées fixées sur un support.

Après avoir établi la courbe de réponse du capteur en fonction du diamètre il est possible de l'extrapoler, par exemple quand le diamètre des réflecteurs étalons les plus petits est de 100 µm, pour de plus petites valeurs du diamètre et de pouvoir ainsi mesurer avec une bonne précision des tailles d'inclusions plus petites, jusqu'à 50 µm et même en dessous.

Les réflecteurs étalons sont typiquement en matière réfractaire et inerte vis à vis du métal liquide, en général de l'alumine dans le cas de l'aluminium; ils peuvent avantageusement être revêtus d'un dépôt (de titane par exemple) pour garantir un bon mouillage par le métal liquide.

L'étape de visualisation des signaux peut être réalisée par la juxtaposition des signaux réfléchis successifs, représentation connue sous le nom d'échographie de type B scan. Il est possible d'utiliser une méthode de type C scan.

On voit qu'en procédant selon l'invention, l'étalonnage est simple à mettre en oeuvre, ne pollue pas le métal liquide et est rigoureux, compte tenu de la connaissance exacte et individuelle du diamètre des réflecteurs étalons et du fait que l'étalonnage peut être effectué aisément à tout moment à titre de contrôle sans affecter la production. Ainsi la précision de la mesure de la taille des inclusions se trouve garantie.

L'invention a également pour objet un dispositif permettant de mettre en oeuvre le procédé d'analyse des inclusions contenues dans un flux de métal liquide selon l'invention. Ce dispositif est caractérisé en ce qu'il comprend un capteur à ultrasons comportant au moins un moyen d'émission (1), tel qu'une sonde émettrice, au moins un moyen de réception (2), tel qu'une sonde réceptrice, et leurs accessoires, au moins un réflecteur étalon, un dispositif d'acquisition et de traitement des échos d'ultrasons réfléchis, un dispositif de visualisation des dites inclusions et un dispositif d'analyse d'image pour les compter et en mesurer le diamètre.

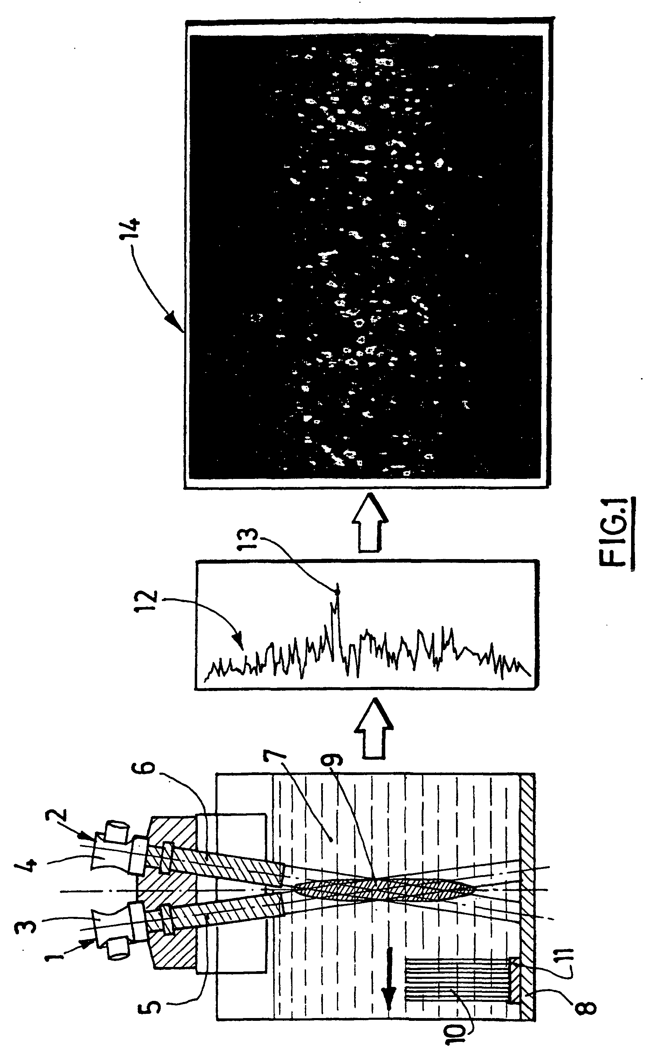

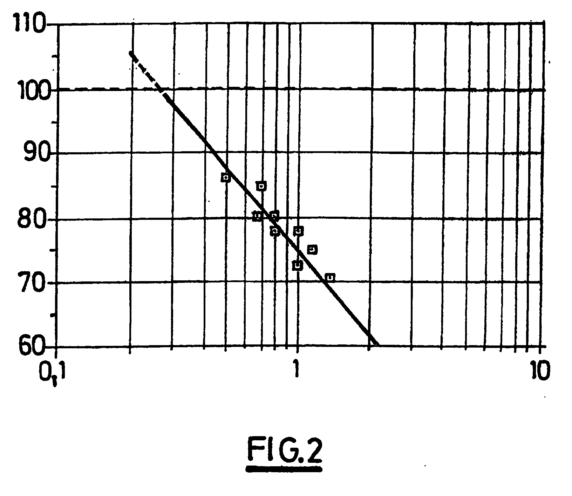

La figure 1 illustre schématiquement l'invention. La figure 2 donne un exemple de courbe d'étalonnage.

Dans la figure 1 on voit que le capteur comprend essentiellement un moyen d'émission (1) et un moyen de réception (2). Dans le mode de réalisation préféré de l'invention, ledit moyen pour émettre ladite succession de tirs de faisceaux d'ultrasons, ou "moyen d'émission", est typiquement une sonde, dite "émettrice", apte à émettre lesdits tirs, et ledit moyen pour recevoir lesdits échos réfléchis par les dites inclusions, ou "moyen de réception", est typiquement une sonde, dite "réceptrice", apte à recevoir lesdits échos. La séparation des fonctions émission et réception permet d'améliorer la sensibilité du capteur. Il est toutefois possible, dans le cadre de l'invention, de regrouper lesdits, ou une partie desdits, moyens d'émission et de réception en une sonde, dite "émettrice/réceptrice", apte à effectuer les fonctions d'émission et de réception. Dans le cas où l'on utilise plus d'un moyen d'émission et/ou plus d'un moyen de réception, il est également possible de combiner des sondes à fonction unique (émission ou réception) et des sondes émettrice/réceptrice.

Chaque moyen d'émission ou de réception comporte au moins un élément traducteur à ultrasons (3, 4) (par exemple comportant des transducteurs de type piézoélectrique) prolongé par une ligne à retard (5, 6) faisant la liaison entre le traducteur (3, 4) et le flux de métal liquide (7) circulant dans une goulotte (8) dans le sens de la flèche.

Les caractéristiques de la ligne à retard sont de bien tenir à la température, d'avoir une bonne inertie chimique par rapport au métal liquide, une transparence élevée aux ultrasons et d'être bien mouillée par le dit métal liquide pour que la transmission du signal se fasse avec le moins de pertes possibles. Les matériaux convenant pour cette application sont généralement des métaux tels que Fe, Al, Zr, Ti, Nb ou des céramiques comme TiB2, quartz, carbure, nitrure, oxycarbure, oxycarbonitrure (SiAlON). Quand la ligne à retard est du même métal que le métal liquide analysé, il convient de la refroidir pour éviter sa destruction.

L'intersection des faisceaux émis et reçus respectivement par les moyens d'émission (1) et de réception (2) forment une tache focale (9) qui est le volume à l'intérieur duquel l'étalonnage est fait et les inclusions analysées. La tache focale peut intéresser toute la hauteur de la veine de métal liquide dans la goulotte et son volume peut représenter jusqu'à 10% du volume du métal circulant dans la goulotte, voire davantage, jusqu'à la totalité, selon la forme de la goulotte.

Le réflecteur étalon de la figure comprend un jeu de tiges réfractaires inertes (10) de diamètres variés, généralement supérieurs à 100 µm, mais connus; les dites tiges sont typiquement fixées verticalement sur un support mobile (11) posé sur le fond de la goulotte (8). Lors de l'étape d'étalonnage ou des contrôles épisodiques effectués au cours des séquences de mesure, le support est déplacé de façon à amener les tiges (10) dans la tache focale (9) puis à les en retirer.

A l'aide de ce dispositif on émet des tirs successifs d'ultrasons. La fréquence des ultrasons est choisie généralement dans une gamme de 0,5 à 50 MHz, et de préférence de 5 à 50 MHz, ce qui correspond, dans la plage de préférence, à une limite de détection typique des particules (λ/10) comprise respectivement entre 100 et 10 µm. La fréquence des tirs est habituellement d'environ de 1 kHz.

L'étape d'acquisition et de traitement des signaux est réalisée informatiquement et conduit à chaque tir à un diagramme (12) comportant un bruit de fond et des pics (13) correspondants à des réflexions sur les obstacles réflecteurs (les inclusions et/ou les réflecteurs étalons (10)) situés dans la tache focale (9). Quand la dite tache focale (9) occupe toute la hauteur de la veine de métal liquide, on note des pics correspondants aux échos du fond de la goulotte.

Comme cela a été déjà dit, l'amplitude du signal d'écho obtenu dans ce diagramme varie avec le diamètre de l'obstacle réflecteur pour une fréquence d'ultrasons donnée. A titre d'illustration la figure 2 montre une courbe détalonnage obtenue à l'aide des réflecteurs étalons (10) de diamètres variés et donnant l'amplitude du signal reçu (en ordonnée) en fonction du diamètre des obstacles réflecteurs (en abscisse). Cette courbe permet une extrapolation aisée du côté des petits diamètres dans le cas où on ne disposerait pas d'étalonnage dans cette zone.

Les informations correspondant aux diagrammes successifs (12) sont ensuite traitées informatiquement dans l'étape de visualisation à l'aide d'une représentation du type B scan pour obtenir une image (14) des échos des obstacles réflecteurs, glissante à la vitesse de la veine de métal liquide, sur laquelle les dits échos sont représentées par des taches dont l'intensité est liée à la taille des dits obstacles. On voit qu'après avoir étalonné ces taches à l'aide des réflecteurs étalons calibrés (10), il est aisé de compter et mesurer le diamètre des inclusions de façon précise à l'aide d'une analyse d'image.

### Exemple

Une caractérisation a été réalisée sur une ligne de coulée expérimentale et la qualité du métal liquide issu de deux fours a été évaluée. L'un des fours était propre, l'autre était contaminé en inclusions. Le nombre des pics correspondant à des inclusions de taille équivalente supérieure à 300 et 500 µm, qui a été estimé à partir du volume sondé (environ 5 % du volume coulé) et exprimé en nombre par kg, est reporté dans le tableau 1.

**Tableau 1**

| | Four contaminé | Four propre |
|---|---|---|
| Inclusions de taille équivalente supérieure à 300 µm | 0,40 | 0,03 |
| Inclusions de taille équivalente supérieure à 500 µm | 0,12 | 0,00 |

### Avantages de l'invention

Le procédé est surtout intéressant pour compter de façon précise les plus grosses inclusions typiquement supérieures à 100 µm qui sont généralement peu nombreuses, donc difficiles à repérer par les autres méthodes d'analyse, mais qui sont les plus nuisibles, et en déterminer la taille.

La présente invention permet avantageusement d'analyser la totalité d'un flux de métal liquide circulant dans un récipient ou une goulotte. Pour cela il suffit de disposer plusieurs moyens d'émission et de réception, typiquement des sondes, avec leur ligne à retard, côte à côte ou en quinconce en travers du flux de métal liquide ou encore de disposer d'un capteur de la largeur du dit flux dans lequel chaque moyen d'émission et de réception comporte un traducteur multi-éléments comportant une pluralité de transducteurs piézoélectriques contigus prolongés par une ligne à retard également adaptée à la largeur du flux de métal liquide. Dans tous les cas la pluralité de traducteurs est associée à une commande électronique permettant le balayage électronique du faisceau acoustique ainsi constitué. De préférence, ladite commande permet également la focalisation du faisceau acoustique. La totalité du flux de métal liquide est analysé dans le mesure où la tache focale traverse toute le veine de métal liquide dans le sens vertical.

## Revendications

1. Procédé de visualisation, de mesure de la taille et de comptage individuels des inclusions en suspension dans un métal liquide en mouvement (7), à l'aide d'un capteur comprenant au moins un moyen (1) pour émettre une succession de tirs de faisceaux d'ultrasons au sein du dit métal liquide, au moins un moyen (2) pour recevoir les échos réfléchis par les dites inclusions, et leurs accessoires, **caractérisé en ce qu'**il comporte une étape d'étalonnage de la réponse du capteur, la dite étape mettant en oeuvre au moins un réflecteur étalon (10) de dimension connue stable au cours du temps, des étapes successives d'acquisition et de traitement des échos réfléchis, une étape de visualisation et un étape d'analyse d'image pour compter et mesurer le diamètre des inclusions.

2. Procédé selon la revendication 2, **caractérisé en ce que** le ou chaque réflecteur étalon est une tige.

3. Procédé selon la revendication 3, **caractérisé en ce qu'**une extrémité de ladite tige est à face plane.

4. Procédé selon la revendication 1, **caractérisé en ce que** l'étape d'étalonnage est effectuée en introduisant une pluralité de réflecteurs étalons, inertes vis à vis du métal liquide, de diamètres calibrés dans la tache focale (9) formée par l'intersection des faisceaux d'ultrasons émis et reçus par lesdits moyens (1, 2).

5. Procédé selon la revendication 4, **caractérisé en ce que** ladite pluralité de réflecteurs étalons est un jeu de tiges.

6. Procédé selon la revendication 5, **caractérisé en ce qu'**au moins l'extrémité desdites tiges immergée dans ladite tache focale est à face plane.

7. Procédé selon l'une quelconque des revendications 1 à 6, **caractérisé en ce que** les ultrasons ont une fréquence comprise entre 0,5 et 50 MHz, et de préférence entre 5 et 50 MHz.

8. Dispositif permettant de mettre en oeuvre le procédé d'analyse des inclusions contenues dans un flux de métal liquide de l'une quelconque des revendications 1 à 7, **caractérisé en ce qu'**il comprend un capteur à ultrasons comportant au moins un moyen d'émission (1), au moins un moyen de réception (2), et leurs accessoires, au moins un réflecteur étalon, un dispositif d'acquisition et de traitement des échos d'ultrasons réfléchis, un dispositif de visualisation des dites inclusions et un dispositif d'analyse d'image pour les compter et en mesurer le diamètre.

9. Dispositif selon la revendication 8, **caractérisé en ce que** le ou chaque réflecteur étalon comprend une tige de diamètre connu.

10. Dispositif selon la revendication 9, **caractérisé en ce qu'**une extrémité de ladite tige est à face plane.

11. Dispositif selon la revendication 8, **caractérisé en ce que** ledit au moins un réflecteur étalon comprend un jeu de tiges de diamètres connus.

12. Dispositif selon la revendication 11, **caractérisé en ce que** lesdites tiges ont un diamètre différent.

13. Dispositif selon la revendication 11 ou 12, **caractérisé en ce qu'**une extrémité desdites tiges est à face plane.

14. Dispositif selon l'une quelconque des revendications 8 à 13, **caractérisé en ce que** le, ou chaque, moyen d'émission est une sonde émettrice et **en ce que** le, ou chaque, moyen de réception est une sonde réceptrice.

15. Dispositif selon l'une quelconque des revendications 8 à 13, **caractérisé en ce que** ledit au moins un moyen d'émission et ledit au moins un moyen de réception sont regroupés en une sonde émettrice/réceptrice apte à effectuer les fonctions d'émission et de réception.

16. Dispositif selon l'une quelconque des revendications 8 à 15, **caractérisé en ce qu'**il comprend des moyens de capteurs à ultrasons pour sonder la totalité de la largeur du flux de métal liquide.

17. Dispositif selon la revendication 16, **caractérisé en ce qu'**il comporte plusieurs moyens d'émission et de réception, avec leur ligne à retard, côte à côte ou en quinconce en travers du flux de métal liquide.

18. Dispositif selon la revendication 16, **caractérisé en ce qu'**il comporte un capteur de la largeur du dit flux dans lequel chaque moyen d'émission et de réception comporte un traducteur multi-éléments comportant une pluralité de transducteurs piézoélectriques contigus prolongés par une ligne à retard également adaptée à la largeur du flux de métal liquide.

19. Dispositif selon la revendication 18, **caractérisé en ce qu'**il comporte une commande électronique d'émission et réception permettant de balayer et de focaliser le faisceau acoustique.

## Claims

1. Process for displaying, measuring the size and counting individual inclusions in suspension in a moving liquid metal (7), using a sensor comprising at least one means (1) of emitting a series of ultrasound beam pulses within the said liquid metal, at least one means (2) of receiving echoes reflected by the said inclusions, and their accessories, **characterized in that** it comprises a sensor response calibration step, the said step comprising using at least one control reflector (10) with known dimensions and stable with time, successive steps for the acquisition and processing of reflected echoes, a display step and an image analysis step to count and measure the diameter of inclusions.

2. Process according to claim 2, **characterized in that** each control reflector is a rod.

3. Process according to claim 3, **characterized in that** the end of the said rod has a flat surface.

4. Process according to claim 1, **characterized in that** the calibration step is carried out by introducing plurality of control reflectors that are inert with respect to the liquid metal, with calibrated diameters in the focal spot (9) formed by the intersection of ultrasound beams emitted and received by the said means (1, 2).

5. Process according to claim 4, **characterized in that** the said plurality of control reflectors is a set of rods.

6. Process according to claim 5, **characterized in that** at least the end of the said rods immersed in the said focal spot has a flat surface.

7. Process according to any one of claims 1 to 6, **characterized in that** the frequency of the ultrasounds is between 0.5 and 50 MHz, and preferably between 5 and 50 MHz.

8. Device for implementing the process for analysis of inclusions contained in a liquid metal flow according to any one of claims 1 to 7, **characterized in that** it comprises an ultrasound sensor comprising at least one emission means (1), at least one reception means (2) and their accessories, at least one control reflector, a reflected ultrasounds echo acquisition and processing device, a display device for the said inclusions and an image analysis device to count them and measure their diameter.

9. Device according to claim 8, **characterized in that** each control reflector has a rod with a known diameter.

10. Device according to claim 9, **characterized in that** the end of the said rod has a flat surface.

11. Device according to claim 8, **characterized in that** the said at least one control reflector comprises a set of rods with known diameters.

12. Device according to claim 11, **characterized in that** the diameters of the said rods are different.

13. Device according to claim 11 or 12, **characterized in that** one end of the said rods has a flat surface.

14. Device according to any one of claims 8 to 13, **characterized in that** each emission means is an emitting probe and that each reception means is a receiving probe.

15. Device according to any one of claims 8 to 13, **characterized in that** the said at least one emission means and the said at least one reception means are grouped in an emission/reception probe capable of performing the emission and reception functions.

16. Device according to any one of claims 8 to 15, **characterized in that** it comprises ultrasound sensor means to probe the entire width of the liquid metal flow.

17. Device according to claim 16, **characterized in that** it comprises several emission and reception means with their delay line, side by side or staggered through the liquid metal flow.

18. Device according to claim 16, **characterized in that** it comprises a sensor having the width of said flow and in which each emission and reception means comprises a multi-element translator comprising several contiguous piezoelectric transducers prolonged by a delay line that is also adapted to the flow width of the liquid metal.

19. Device according to claim 18, **characterized in that** it comprises an electronic emission and reception control to scan and focus the acoustic beam.

## Patentansprüche

1. Verfahren zur individuellen Visualisierung, Größenmessung und Zählung suspendierter Einschlüsse in einem bewegten Flüssigmetall (7) mittels eines Sensors, welcher mindestens ein Mittel (1) zum Senden einer Folge von Ultraschall-Strahlenschüssen ins Innere des Flüssigmetalls, mindestens ein Mittel (2) zum Empfang der von den Einschlüssen reflektierten Echos sowie ihre Zubehöre aufweist, **dadurch gekennzeichnet, dass** es einen Schritt zur Eichung der Sensorantwort, bei dem mindestens ein zeitlich stabiler Eichreflektor (10) bekannter Größe eingesetzt wird, sukzessive Schritte zur Erfassung und Behandlung der reflektierten Echos, einen Visualisierungsschritt und einen Bildanalyseschritt zum Zählen und zum Messen des Durchmessers der Einschlüsse umfasst.

2. Verfahren nach Anspruch 2, **dadurch gekennzeichnet, dass** der bzw. jeder Eichreflektor eine Stange ist.

3. Verfahren nach Anspruch 3, **dadurch gekennzeichnet, dass** ein Ende der Stange ebenflächig ist.

4. Verfahren nach Anspruch 1, **dadurch gekennzeichnet, dass** der Eichschritt durch Einbringen einer Vielzahl von Eichreflektoren erfolgt, die gegenüber dem Flüssigmetall träge sind und im durch die Kreuzung der von den Mitteln (1, 2) gesendeten und empfangenen Ultraschallstrahlen ausgebildeten Brennfleck (9) kalibrierte Durchmesser haben.

5. Verfahren nach Anspruch 4, **dadurch gekennzeichnet, dass** die Vielzahl von Eichreflektoren ein Satz Stangen ist.

6. Verfahren nach Anspruch 5, **dadurch gekennzeichnet, dass** mindestens das im Brennfleck versenkte Ende der Stangen ebenflächig ist.

7. Verfahren nach einem der Ansprüche 1 bis 6, **dadurch gekennzeichnet, dass** die Ultraschallstrahlen eine Frequenz zwischen 0,5 und 50 MHz, vorzugsweise zwischen 5 und 50 MHz haben.

8. Vorrichtung zur Durchführung des Verfahrens zur Analyse der in einem Flüssigmetallstrom enthaltenen Einschlüsse nach einem der Ansprüche 1 bis 7, **dadurch gekennzeichnet, dass** sie einen Ultraschallsensor mit mindestens einem Sendemittel (1), mindestens einem Empfangsmittel (2) und deren Zubehören, mindestens einen Eichreflektor, eine Vorrichtung zur Erfassung und Behandlung der reflektierten Ultraschallechos, eine Vorrichtung zur Visualisierung der Einschlüsse und eine Bildanalysevorrichtung zum Zählen der Einschlüsse und zum Messen ihrer Durchmesser umfasst.

9. Vorrichtung nach Anspruch 8, **dadurch gekennzeichnet, dass** der bzw. jeder Eichreflektor eine Stange bekannten Durchmessers umfasst.

10. Vorrichtung nach Anspruch 9, **dadurch gekennzeichnet, dass** ein Ende der Stange ebenflächig ist.

11. Vorrichtung nach Anspruch 8, **dadurch gekennzeichnet, dass** der mindestens eine Eichreflektor einen Satz Stangen bekannter Durchmesser umfasst.

12. Vorrichtung nach Anspruch 11, **dadurch gekennzeichnet, dass** die Stangen einen unterschiedlichen Durchmesser haben.

13. Vorrichtung nach Anspruch 11 oder 12, **dadurch gekennzeichnet, dass** ein Ende der Stangen ebenflächig ist.

14. Vorrichtung nach einem der Ansprüche 8 bis 13, **dadurch gekennzeichnet, dass** das bzw. jedes Sendemittel eine Sendesonde ist und dass das bzw. jedes Empfangsmittel eine Empfangssonde ist.

15. Vorrichtung nach einem der Ansprüche 8 bis 13, **dadurch gekennzeichnet, dass** das mindestens eine Sendemittel und das mindestens eine Empfangsmittel in einer Sende- und Empfangssonde zusammengefasst sind, die geeignet ist, die Sende- und Empfangsfunktionen auszuführen.

16. Vorrichtung nach einem der Ansprüche 8 bis 15, **dadurch gekennzeichnet, dass** sie Ultraschall-Sensorenmittel aufweist, um die gesamte Breite des Flüssigmetallstroms zu sondieren.

17. Vorrichtung nach Anspruch 16, **dadurch gekennzeichnet, dass** sie mehrere Sende- und Empfangsmittel mit ihrer Verzögerungsleitung nebeneinander oder quer zum Flüssigmetallstrom aufweist.

18. Vorrichtung nach Anspruch 16, **dadurch gekennzeichnet, dass** sie einen Sensor der Breite des Stroms aufweist, bei dem jedes Sende- und Empfangsmittel einen Mehrelement-Umsetzer mit einer Vielzahl von aneinandergrenzenden piezoelektrischen Wandlern enthält, welche durch eine wiederum an die Breite des Flüssigmetallstroms angepasste Verzögerungsleitung verlängert sind.

19. Vorrichtung nach Anspruch 18, **dadurch gekennzeichnet, dass** sie eine elektronische Sende- und Empfangssteuerung zur Abtastung und zur Fokussierung des Schallstrahls aufweist.
